# EUROPEAN PATENT APPLICATION

(11) **EP 4 585 212 A1**
(43) Date of publication of application: **16.07.2025**
(21) Application number: 23863343.2
(22) Date of filing: 26.07.2023
(51) Int. Cl.: A61K 9/51

(54) **KIT FOR MANUFACTURE OF DRUG-CONTAINING NANOPARTICLES AND NANOPARTICLE COMPOSITION FOR DRUG DELIVERY**

(30) Priority: 08.09.2022 KR 20220114547; 08.09.2022 KR 20220114552
(71) Applicant: Samyang Holdings Corporation, Seoul 03129 (KR)
(72) Inventor: KIM, Go Eun, Seongnam-si Gyeonggi-do 13488 (KR); KYUNG, Kyu Jin, Yongin-si Gyeonggi-do 16827 (KR); KIM, So Young, Seongnam-si Gyeonggi-do 13647 (KR); NAM, Joung Pyo, Seoul 02471 (KR); LEE, So Jin, Seoul 02468 (KR); PARK, Jong Min, Seongnam-si Gyeonggi-do 13590 (KR); SEO, Won Il, Seongnam-si Gyeonggi-do 13488 (KR)
(74) Representative: Gleiss Große Schrell und Partner mbB
(86) International application number: PCT/KR2023/010824
(87) International publication number: WO 2024/053862

(57) **Abstract**

The present invention relates to a kit for construction of drug-containing nanoparticles and a nanoparticle composition for drug delivery. More specifically, the present invention concerns a kit for construction of drug-containing nanoparticles and a nanoparticle composition for drug delivery, each designed to increase the efficiency of cellular drug delivery by utilizing nanoparticles that include a cationic compound and an anionic polymer compound with at least one acidic functional group.

## Description

### TECHNICAL FIELD

The present invention relates to a kit for preparing nanoparticles comprising drug and a nanoparticle composition for drug delivery, and more specifically, a kit for preparing drug-containing nanoparticles and a nanoparticle composition for drug delivery designed so that by utilizing nanoparticles comprising cationic compound and anionic polymer compound having at least one acid functional group, the cell delivery efficiency of the drug can be increased.

### BACKGROUND ART

In therapies using anionic drugs including nucleic acid, technologies for safe and efficient drug delivery have been researched for a long time, and various carriers and techniques for delivery have been developed. Carriers are mainly divided into viral carriers utilizing adenovirus, retrovirus or the like, and non-viral carriers utilizing cationic lipid, cationic polymer or the like. Viral carriers are known as being exposed to risks such as non-specific immune response, etc. and having many problems in commercialization due to the complexity of the production process. Thus, recent researches proceed in the direction to improve such disadvantages by using non-viral carriers. In comparison with viral carriers, non-viral carriers have advantages of fewer side effects in terms of *in vivo* safety, and lower production cost in terms of economy.

The representative non-viral carriers for delivering nucleic acid material are a complex of cationic lipid and nucleic acid (lipoplex) and a complex of polycationic polymer and nucleic acid (polyplex). Such a cationic lipid or polycationic polymer stabilizes anionic drug by forming a complex through electrostatic interaction with the anionic drug and increases intracellular delivery, and for these reasons, various researches thereof have been conducted (De Paula D, Bentley MV, Mahato RI, Hydrophobization and bioconjugation for enhanced siRNA delivery and targeting, RNA 13 (2007) 431-56; Gary DJ, Puri N, Won YY, Polymer-based siRNA delivery: Perspectives on the fundamental and phenomenological distinctions from polymer-based DNA delivery, J Control release 121 (2007) 64-73).

Nanoparticles comprising drug frequently lose stability easily according to the storage environment, and thus they are vulnerable to long-term storage and the quality may be damaged during transportation. In addition, since a complicated production process is necessary for securing sufficient stability, the production is very demanding. Therefore, it has been requested to develop a composition for drug delivery, which is not significantly affected by the storage environment and can be easily used by an end user, and can further increase the cell delivery efficiency of the drug.

In addition, recently it has been requested to develop personalized vaccine, and in a pandemic situation, it is important to promptly supply drug-containing nanoparticles to patients with minimal processing time. Therefore, there is a need for a technology that shortens the process period and, furthermore, provides ready-made nanoparticles regardless of the kind of drug.

### CONTENTS OF THE INVENTION

### PROBLEMS TO BE SOLVED

The first aspect of the present invention is to provide a kit for preparing drug-containing nanoparticle, and according to this, the drug can be easily contained in the nanoparticle only by simple mixing of the components of the kit and thus can be easily used by an end user. In addition, the drug-containing nanoparticles can be easily and quickly prepared immediately before the use regardless of the kind of drug (e.g., mRNA) and thus the drug can be effectively delivered into the body without influence according to the storage or transportation environment, and the cell delivery efficiency of the drug can be further increased. In particular, the drug-containing nanoparticle formed by the kit according to the first aspect of the present invention can be specifically delivered to spleen, thereby further increasing the usability of the anticancer vaccine.

In addition, the second aspect of the present invention is to provide a nanoparticle composition for drug delivery, and according to this, the drug-containing nanoparticles can be easily and quickly prepared immediately before the use regardless of the kind of drug (e.g., mRNA), and once the drug is determined, it can be easily contained in the nanoparticles only by simple mixing with the drug, and thus the preparation of drug-containing nanoparticles is simple and convenient for end users to use. Also, since stable and effective drug-containing nanoparticles can be formed even when the drug is changed between antigen mRNAs with different base sequences such as OVA, mTrp2, and hTrp2, etc., various drugs can be applied to the nanoparticles developed as ready-made products, and can be administered to patients with minimal risk of drug denaturation due to less influence from storage or transportation environment, and the cell delivery efficiency of the drug can be further increased. In particular, the drug-containing nanoparticle formed by mixing the drug with the composition according to the first aspect of the present invention can be specifically delivered to spleen, thereby further increasing the usability of the anticancer vaccine.

### TECHNICAL MEANS

The first aspect of the present invention provides a kit for preparing drug-containing nanoparticles, comprising: a first chamber comprising nanoparticles comprising cationic compound and anionic polymer compound; and a second chamber comprising drug which is effective ingredient selected from nucleic acid, polypeptide, virus or combination thereof, wherein the anionic polymer compound has at least one acid functional group.

In an embodiment of the first aspect, the drug-containing nanoparticles are for intracellular delivery of the drug.

In an embodiment of the first aspect, the anionic polymer compound is anionic amphiphilic block copolymer, anionic hydrophilic polymer, anionic hydrophobic polymer, or combination thereof.

In an embodiment of the first aspect, the anionic amphiphilic block copolymer comprises hydrophilic block and hydrophobic block.

In an embodiment of the first aspect, the anionic hydrophilic polymer comprises hydrophilic block only.

In an embodiment of the first aspect, the anionic hydrophobic polymer comprises hydrophobic block only.

In an embodiment of the first aspect, one or more selected from the group consisting of the first chamber and the second chamber further comprise additional solvent.

In an embodiment of the first aspect, the solvent is aqueous solvent, water miscible solvent, or a mixture thereof.

In an embodiment of the first aspect, the second chamber further comprises one or more additives selected from pH adjusting agent, inorganic salt, saccharide, surfactant, chelating agent, or a combination thereof.

In an embodiment of the first aspect, the kit for preparing drug-containing nanoparticles may consist of: the drug which is effective ingredient selected from nucleic acid, polypeptide, virus or combination thereof; the cationic compound; the anionic polymer compound; solvent; and one or more additives selected from pH adjusting agent, inorganic salt, saccharide, surfactant, chelating agent, or a combination thereof.

In an embodiment of the first aspect, the amount of the anionic polymer compound may be 0.01 to 15 parts by weight, based on 1 part by weight of the cationic compound.

In an embodiment of the first aspect, the cationic compound and the anionic polymer compound may be used for the nanoparticle preparation in the form of solution filtered one or more times.

The second aspect of the present invention provides a nanoparticle composition for drug delivery which is a composition for drug delivery comprising nanoparticle, wherein the nanoparticle comprises cationic compound and anionic polymer compound, wherein the nanoparticle does not comprise drug, and wherein the anionic polymer compound has at least one acid functional group.

In an embodiment of the second aspect, the nanoparticle composition for drug delivery is for intracellular delivery of drug.

In an embodiment of the second aspect, the drug is selected from nucleic acid, polypeptide, virus or combination thereof.

In an embodiment of the second aspect, the anionic polymer compound is anionic amphiphilic block copolymer, anionic hydrophilic polymer, anionic hydrophobic polymer, or combination thereof.

In an embodiment of the second aspect, the anionic amphiphilic block copolymer comprises hydrophilic block and hydrophobic block.

In an embodiment of the second aspect, the anionic hydrophilic polymer comprises hydrophilic block only.

In an embodiment of the second aspect, the anionic hydrophobic polymer comprises hydrophobic block only.

In an embodiment of the second aspect, the nanoparticle composition for drug delivery further comprises additional solvent.

In an embodiment of the second aspect, the solvent is aqueous solvent, water miscible solvent, or a mixture thereof.

In an embodiment of the second aspect, the nanoparticle composition for drug delivery further comprises one or more additives selected from pH adjusting agent, inorganic salt, saccharide, surfactant, chelating agent, or a combination thereof.

In an embodiment of the second aspect, the nanoparticle composition for drug delivery may consist of: the cationic compound; the anionic polymer compound; solvent; and one or more additives selected from pH adjusting agent, inorganic salt, saccharide, surfactant, chelating agent, or a combination thereof.

In an embodiment of the second aspect, the amount of the anionic polymer compound may be 0.01 to 15 parts by weight, based on 1 part by weight of the cationic compound.

In an embodiment of the second aspect, the cationic compound and the anionic polymer compound may be used for the nanoparticle preparation in the form of solution filtered one or more times.

### EFFECT OF THE INVENTION

In the kit for preparing drug-containing nanoparticles according to the first aspect of the present invention, the drug and the nanoparticles are isolated from each other and contained in separate chambers, and thus there is no influence by the storage or transportation environment, and when using the kit, an end user can quickly prepare drug-containing nanoparticles regardless of the kind of drug (e.g., mRNA) only by simple mixing of the components of the kit without complicated process, and accordingly, for example, in case of personalized vaccine or pandemic situation, once the therapeutic mRNA is produced, it can be simply mixed with the nanoparticles suggested in the present invention and promptly administered into human body, even without production process optimization with the mRNA. In addition, the kit for preparing drug-containing nanoparticles according to the present invention can further increase the cell delivery efficiency of the drug, as compared with a kit comprising amphiphilic polymer. In particular, the drug-containing nanoparticle formed by the kit for preparing drug-containing nanoparticles of the present invention can be specifically delivered to spleen, thereby further increasing the usability of the anticancer vaccine.

In addition, the nanoparticle composition for drug delivery according to the second aspect of the present invention is not influenced by the storage or transportation environment, and when using it, an end user can quickly prepare drug-containing nanoparticles regardless of the kind of drug (e.g., mRNA) only by simple mixing of the composition and the drug without complicated process, and accordingly, for example, since there is no need of production process optimization with the mRNA even in case of personalized vaccine in hospital or pandemic situation, once the final mRNA is produced, it can be simply mixed with the composition of the present invention and promptly administered into human body. In addition, the nanoparticle composition for drug delivery according to the present invention can further increase the cell delivery efficiency of the drug, as compared with a nanoparticle composition comprising amphiphilic polymer. In particular, the drug-containing nanoparticle formed by mixing the drug with the composition according to the first aspect of the present invention can be specifically delivered to spleen, thereby further increasing the usability of the anticancer vaccine.

### BRIEF EXPLANATION OF THE DRAWINGS

Figure 1 is an image showing the result of agarose gel electrophoresis in the experiment to confirm mRNA-containing nanoparticle preparation conducted in Example 1 of the present invention.
Figure 2 is an image showing the results of agarose gel electrophoresis in the experiment to confirm mRNA-containing nanoparticle preparation conducted in Examples 2 to 5 and Comparative Examples 1 to 3 of the present invention.

### CONCRETE MODE FOR CARRYING OUT THE INVENTION

The present invention will be explained in detail below.

### [The first aspect: Kit for preparing drug-containing nanoparticles]

The kit for preparing drug-containing nanoparticles according to the first aspect of the present invention comprises: a first chamber comprising nanoparticles comprising cationic compound and anionic polymer compound; and a second chamber comprising drug which is effective ingredient selected from nucleic acid, polypeptide, virus or combination thereof, wherein the anionic polymer compound has at least one acid functional group.

The kit of the present invention consists of two or more chambers, and an end user can easily form drug-containing nanoparticles only by simple mixing of the contents in the chambers. The term "simple mixing" can include all actions of "mixing," and it means that the action of mixing is not subject to any specific conditions. The mixing can be done in various manners such as dropping, vortexing, decanting, etc., but it is not limited thereto. According to an embodiment, in case of using the kit of the present invention, drug-containing nanoparticles can be formed rapidly, for example, within 1 minute, within 30 seconds, or within 15 seconds, in an amount of 90% or more, 95% or more, or 99% or more of the amount that can be formed theoretically.

In an embodiment, the cationic compound and the anionic polymer compound form nanoparticles through electrostatic interaction, and an end user can form drug-containing nanoparticles by simply mixing the formed nanoparticles and the drug. Thus, according to an embodiment, the drug-containing nanoparticles prepared by the kit of the present invention can be in a form where at least a part of the drug is combined to the outside of the nanoparticle. Such a drug-containing nanoparticle structure improves stability of the drug in blood or body fluid.

The "nucleic acid" can be, for example, DNA, RNA, siRNA, shRNA, miRNA, mRNA, aptamer, antisense oligonucleotide, or a combination thereof, but it is not limited thereto.

The "polypeptide" may mean a protein having activity in the body such as antibody or fragment thereof, cytokine, hormone or analog thereof, or a protein that can be recognized as antigen through a series of processes in the body, including polypeptide sequence of antigen, analog or precursor thereof.

The "virus" may be oncolytic virus, and for example, may be one or more selected from the group consisting of adenovirus, vaccinia virus, herpes simplex virus (HSV) and vesicular stomatitis virus (VSV). In an embodiment, the oncolytic virus is adenovirus. The adenovirus used in the concrete example of the present invention comprises luciferase gene, and it can be confirmed through imaging.

The virus can express several kinds of therapeutic genes in the body of the subject, and it is not limited in terms of specific molecular weight, protein, bioactivity or field of therapy. The virus for prevention can induce immunity to the target disease in the body of the subject. Nanoparticles comprising virus for preventing disease can reduce immunity induction due to the virus itself, designate or extend the target cell, and reduce hyperimmune reaction to the virus when administered again and thereby provide advantage of obtaining available effect by inoculation for several times.

In an embodiment, the particle size of the nanoparticles can be defined by Z-average value, and for example, it may be 800 nm or less, 600 nm or less, 500 nm or less, 400 nm or less, 300 nm or less, 200 nm or less, or 180 nm or less, and also may be 10 nm or more, 50 nm or more, or 100 nm or more. In a concrete embodiment, the particle size of the nanoparticles defined by Z-average value may be, for example, 10 to 800 nm, 10 to 600 nm, 10 to 500 nm, or 10 to 400 nm.

The "Z-average" may mean the average of hydrodynamic diameter of particle distribution measured by using Dynamic Light Scattering (DSL). The nanoparticles have a monodisperse particle distribution, and the polydispersity index thereof may be, for example, 0.05 to 0.8, 0.1 to 0.7, or 0.2 to 0.6.

Also, in an embodiment, the surface charge of the nanoparticles may be, for example, -50 mV or more, -45 mV or more, -40 mV or more, or -35 mV or more, and also may be 40 mV or less, 30 mV or less, 20 mV or less, 10 mV or less, or 0 mV or less. In a concrete embodiment, the surface charge of the nanoparticles may be, for example, -50 to 40 mV, -45 to 30 mV, -40 to 20 mV, -40 to 10 mV, or -35 to 0 mV. The surface charge may be measured in an environment close to biological environment, and for example, it may be measured in 8 to 12 mM HEPES buffer (pH 7.0 to 7.5).

In case of maintaining the particle size and surface charge of the nanoparticles to the above level, it is preferable in terms of stability of nanoparticle structure, amounts of the components and absorbability in the body, and easiness of sterilization. For example, in case where the drug is nucleic acid, the one or more ends of the nucleic acid may be modified with one or more selected from the group consisting of cholesterol, tocopherol and fatty acids having 10 to 24 carbon atoms. The cholesterol, tocopherol and fatty acids having 10 to 24 carbon atoms include each analogue, derivative and metabolite of the cholesterol, tocopherol and fatty acids.

The amount of the drug may be, based on the total weight of the drug-containing nanoparticle prepared by the kit of the present invention, for example, 30 % by weight or less, 20 % by weight or less, 10 % by weight or less, 5 % by weight or less, or 1 % by weight or less, and also may be 0.001 % by weight or more, 0.01 % by weight or more, 0.05 % by weight or more, 0.1 % by weight or more, or 0.15 % by weight or more. In a concrete embodiment, the amount of the drug may be, based on the total weight of the drug-containing nanoparticle, for example, 0.001 to 30 % by weight, 0.01 to 20 % by weight, 0.05 to 10 % by weight, 0.1 to 5 % by weight, or 0.15 to 1 % by weight. If the amount of the drug based on the total weight of the drug-containing nanoparticle is less than the above range, the amount of nanoparticle used as delivery carrier becomes too much as compared with the drug, and thus there may be a side effect due to the nanoparticle delivery carrier. If the amount of the drug is greater than the above range, the size of the drug-containing nanoparticle becomes too large, and thus the particle stability may be lowered and the rate of loss during filter sterilization may increase. In case where the drug is virus, the drug-containing nanoparticle may comprise virus 1x10⁶ to 1x10¹⁴ VP (Virus particle), 1x10⁷ to 1x10¹³ VP, 1x10⁸ to 1x10¹² VP, or 1x10⁹ to 1x10¹¹ VP.

In a concrete embodiment, the cationic compound may be cationic lipid or cationic polymer, and more concretely, it may be cationic lipid.

In an embodiment, the cationic lipid may may be one or a combination of two or more selected from the group consisting of N,N-dioleyl-N,N-dimethylammoniumchloride (DODAC), N,N-distearyl-N,N-dimethylammoniumbromide (DDAB), N-(1-(2,3-dioleoyloxy)propyl-N,N,N-trimethylammoniumchloride (DOTAP), N,N-dimethyl-(2,3-dioleoyloxy)propylamine (DODMA), N,N,N-trimethyl-(2,3-dioleoyloxy)propylamine (DOTMA), 1,2-diacyl-3-trimethylammonium-propane (TAP), 1,2-diacyl-3-dimethylammonium-propane (DAP), 3β-[N-(N',N',N'-trimethylaminoethane)carbamoyl]cholesterol (TC-cholesterol), 3β-[N-(N',N'-dimethylaminoethane)carbamoyl]cholesterol (DC-cholesterol), 3β-[N-(N'-monomethylaminoethane)carbamoyl]cholesterol (MC-cholesterol), 3β-[N-(aminoethane)carbamoyl]cholesterol (AC-cholesterol), cholesteryloxypropane-1-amine (COPA), N-(N'-aminoethane)carbamoylpropanoic tocopherol (AC-tocopherol) and N-(N'-methylaminoethane)carbamoylpropanoic tocopherol (MC-tocopherol).

In case of using such a cationic lipid, it is preferable to use polycationic lipid having high cation density in a molecule as less as possible in order to decrease toxicity induced by the cationic lipid, and more concretely, it is preferable to use polycationic lipid wherein the number of the functional group capable of exhibiting positive charge in an aqueous solution is one per molecule.

Accordingly, in a more preferable embodiment, the cationic lipid may be one or more selected from the group consisting of 3β-[N-(N',N',N'-trimethylaminoethane)carbamoyl]cholesterol (TC-cholesterol), 3β-[N-(N',N'-dimethylaminoethane)carbamoyl]cholesterol (DC-cholesterol), 3β-[N-(N'-monomethylaminoethane)carbamoyl]cholesterol (MC-cholesterol), 3β-[N-(aminoethane)carbamoyl]cholesterol (AC-cholesterol), N-(1-(2,3-dioleoyloxy)propyl-N,N,N-trimethylammoniumchloride (DOTAP), N,N-dimethyl-(2,3-dioleoyloxy)propylamine (DODMA) and N,N,N-trimethyl-(2,3-dioleoyloxy)propylamine (DOTMA).

On the other hand, in an embodiment, the cationic polymer may be selected from the group consisting of chitosan, glycol chitosan, protamine, polylysine, polyarginine, polyamidoamine (PAMAM), polyethylenimine, dextran, hyaluronic acid, albumin, high molecular weight polyethylenimine (PEI), polyamine and polyvinylamine (PVAm), and more concretely, it may be one or more selected from the group consisting of polyethylenimine (PEI), polyamine and polyvinylamine (PVAm).

In a concrete embodiment, the cationic lipid may be a cationic lipid of the following Formula 1:

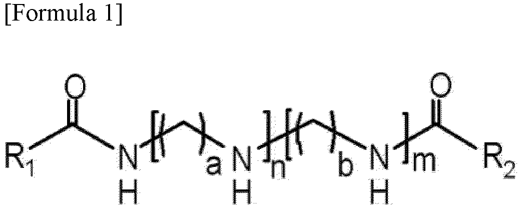

In the above formula,
each of n and m is independently 0 to 12 with the proviso that 2 ≤ n + m ≤ 12;
each of a and b is independently 1 to 6; and
each of R₁ and R₂ is independently selected from the group consisting of saturated and unsaturated hydrocarbon groups having 11 to 25 carbon atoms.

More concretely, in the above formula 1, each of n and m may be independently 1 to 9 with the proviso that 2 ≤ n + m ≤ 10.

More concretely, in the above formula 1, each of a and b may be independently 2 to 4.

More concretely, in the above formula 1, each of R₁ and R₂ may be independently selected from the group consisting of lauryl, myristyl, palmityl, stearyl, arachidyl, behenyl, lignoceryl, cerotyl, myristoleyl, palmitoleyl, sapienyl, oleyl, linoleyl, arachidonyl, eicosapentaenyl, erucyl, docosahexaenyl and cerotyl.

In an embodiment, the cationic lipid may be one or more selected from the group consisting of 1,6-dioleoyl triethylenetetramide [N,N'-((ethane-1,2-diylbis(azanediyl))bis(ethane-2,1-diyl))dioleamide; dioTETA], 1,8-dilinoleoyl tetraethylenepentamide [(9Z,9'Z,12Z,12'Z)-N,N'-(((azanediylbis(ethane-2,1-diyl))bis(azanediyl))bis(ethane-2,1-diyl))bis(octadeca-9,12-dienamide)], 1,4-dimyristoleoyl diethylenetriamide [(9Z,9'Z)-N,N'-(azanediylbis(ethane-2,1-diyl))bis(tetradec-9-enamide)], 1,10-distearoyl pentaethylenehexamide [N,N'-(3,6,9,12-tetraazatetradecane-1,14-diyl)distearamide], and 1,10-dioleoyl pentaethylenehexamide [N,N'-(3,6,9,12-tetraazatetradecane-1,14-diyl)dioleamide].

The amount of the cationic compound in the drug-containing nanoparticle prepared by the kit of the present invention may be, based on 1 part by weight of the drug, for example, 25 parts by weight or less, 20 parts by weight or less, 15 parts by weight or less, 10 parts by weight or less, or 5 parts by weight or less, and also may be 0.5 part by weight or more, 1 part by weight or more, 1.5 parts by weight or more, 2 parts by weight or more, or 2.5 parts by weight or more. In an embodiment, the amount of the cationic compound in the drug-containing nanoparticle may be, based on 1 part by weight of the drug, 0.5 to 25 parts by weight, 1 to 20 parts by weight, 1.5 to 15 parts by weight, 2 to 10 parts by weight, or 2.5 to 5 parts by weight. Also, in case where the drug is virus, more concretely adenovirus, the amount of the cationic compound may be, based on virus 1x10¹⁰ VP, 1 µg or more, 5 µg or more, 10 µg or more, 15 µg or more, or 18 µg or more, and also may be 150 µg or less, 100 µg or less, 50 µg or less, or 30 µg or less, and for example, it may be 1 µg to 150 µg, 5 µg to 100 µg, 10 µg to 50 µg, or 15 µg to 30 µg. If the amount of the cationic compound in the drug-containing nanoparticle is less than the above range, the drug may not be contained stably in the nanoparticle. If the amount of the cationic compound is greater than the above range, the size of the drug-containing nanoparticle becomes too large, and thus the particle stability may be lowered and the rate of loss during filter sterilization may increase.

In case where the drug is nucleic acid, the cationic compound and the nucleic acid are combined together through electrostatic interaction. In an embodiment, the ratio of quantities of electric charge of the cationic compound (N) and the nucleic acid (P) (N/P: the ratio of the positive electric charge of the cationic compound to the negative electric charge of the nucleic acid) may be 0.5 or more, 0.7 or more, 0.9 or more, or 1 or more, and also may be 100 or less, 50 or less, 20 or less, or 10 or less, and for example, it may be 0.5 to 100, 0.7 to 50, 0.9 to 20, or 1 to 10. If the ratio (N/P) is less than the above range, a sufficient amount of the nucleic acid may not be contained in the nanoparticle. If the ratio (N/P) is greater than the above range, toxicity may be induced. In addition, the N/P ratio may act importantly for expression of the effective ingredient specifically in spleen.

In an embodiment, the anionic polymer compound may be anionic amphiphilic block copolymer, anionic hydrophilic polymer, anionic hydrophobic polymer, or combination thereof.

In an embodiment, the anionic amphiphilic block copolymer may be a block copolymer (for example, an A-B type block copolymer comprising a hydrophilic A block and a hydrophobic B block), wherein the hydrophilic block or hydrophobic block has at least one acid functional group, and according to an embodiment, the hydrophobic block may have at least one acid functional group. In an aqueous solution, such a block copolymer forms core-shell type polymer nanoparticle wherein the hydrophobic block forms the core (inner wall) and the hydrophilic block forms the shell (outer wall).

In an embodiment, the anionic hydrophilic polymer is a polymer comprising hydrophilic block only, wherein the hydrophilic block has at least one acid functional group. In an aqueous solution, such a hydrophilic polymer forms shell type polymer nanoparticle wherein the acid functional group electrostatically interacts with the cationic lipid and the hydrophilic block forms the shell (outer wall).

In an embodiment, the anionic hydrophobic polymer is a polymer comprising hydrophobic block only, wherein the hydrophobic block has at least one acid functional group. Such a hydrophobic polymer forms core type polymer nanoparticle wherein the acid functional group of the hydrophobic polymer electrostatically interacts with the cationic lipid and the hydrophobic block forms the core (inner wall).

In an embodiment, the hydrophilic block may be one or more selected from the group consisting of polyalkylene glycol, polyvinyl alcohol, polyvinylpyrrolidone, polyoxazoline, polyacrylamide, and derivatives thereof.

More concretely, the hydrophilic block may be one or more selected from the group consisting of monomethoxypolyethylene glycol (mPEG), monoacetoxypolyethylene glycol, polyethylene glycol, polyethyloxazoline (PEOz), polymethyloxazoline (PMOz), copolymer of polyethylene and propylene glycol, and polyvinylpyrrolidone.

In an embodiment, the number average molecular weight (g/mol) of the hydrophilic block may be 200 or more, 500 or more, 1,000 or more, or 1,500 or more, and it also may be 50,000 or less, 20,000 or less, 10,000 or less, or 5,000 or less, but it is not limited thereto.

Also, if necessary, the end of the hydrophilic block may be chemically combined with a functional group or ligand capable of reaching specific tissue or cell, or a functional group capable of promoting intracellular delivery, in order to control *in vivo* distribution of polymer nanoparticle carrier or to increase the efficiency of delivering the nanoparticle carrier into cell. In an embodiment, the functional group or ligand may be one or more selected from the group consisting of monosaccharides, polysaccharides, vitamins, peptides, proteins, and antibodies to cell surface receptors. More concretely, the functional group or ligand may be one or more selected from the group consisting of anisamide, vitamin B9 (folic acid), vitamin B12, vitamin A, galactose, lactose, mannose, hyaluronic acid, RGD peptide, NGR peptide, transferrin, antibody to transferrin receptor, etc.

The hydrophobic block is a biocompatible, biodegradable polymer, and in an embodiment, it may be one or more selected from the group consisting of polyester, polyanhydride, polyamino acid, polyorthoester and polyphosphazine.

More concretely, the hydrophobic block may be one or more selected from the group consisting of polylactide (PLA), polyglycolide, polycaprolactone (PCL), polydioxan-2-one, copolymer of polylactide and polyglycolide, copolymer of polylactide and polydioxan-2-one, copolymer of polylactide and polycaprolactone, and a copolymer of polyglycolide and polycaprolactone.

In an embodiment, the number average molecular weight (g/mol) of the hydrophobic block may be 200 or more, 500 or more, 700 or more, or 1,000 or more, and it also may be 50,000 or less, 20,000 or less, 10,000 or less, or 6,000 or less, but it is not limited thereto.

Also, in an embodiment, the hydrophobic block may be modified by chemically combining the hydroxyl group at the end thereof with tocopherol, cholesterol, or fatty acid having 10 to 24 carbons.

The hydrophilic block may not have acid functional group or may have at least one acid functional group, and also the hydrophobic block may not have acid functional group or may have at least one acid functional group, but at least one of the hydrophilic block and the hydrophobic block has at least one acid functional group.

In an embodiment, the acid functional group may be derived from an acid selected from the group consisting of inorganic acid, sulfonic acid, carboxylic acid and combinations thereof.

More concretely, the inorganic acid may be selected from the group consisting of phosphoric acid, nitric acid, chromic acid, and combinations thereof, the sulfonic acid may be selected from the group consisting of methanesulfonic acid, ethanesulfonic acid, benzenesulfonic acid, and combinations thereof, and the carboxylic acid may be selected from the group consisting of acetic acid, citric acid, gluconic acid, lactic acid, oxalic acid, tartaric acid, succinic acid, maleic acid, phthalic acid, and combinations thereof, but it is not limited thereto.

In a preferable embodiment, the anionic amphiphilic block copolymer is an A-B type copolymer consisting of a hydrophilic block of monomethoxypolyethylene glycol (number average molecular weight: 300 to 10,000 g/mol) and a hydrophobic block of polylactide (number average molecular weight: 300 to 10,000 g/mol) or polycaprolactone (number average molecular weight: 300 to 10,000 g/mol), and the hydrophobic block may have a carboxylic acid group derived from succinic acid.

In a preferable embodiment, the anionic hydrophilic polymer is a polymer consisting of a hydrophilic block of monomethoxypolyethylene glycol (number average molecular weight: 300 to 10,000 g/mol), polymethyloxazoline (number average molecular weight: 300 to 10,000 g/mol), or polyethyloxazoline (number average molecular weight: 300 to 10,000 g/mol), and it may have a carboxylic acid group derived from succinic acid.

In an embodiment, the molar ratio of the anionic polymer compound to 1 mole of the cationic compound contained in the first chamber may be 0.01 or more, 0.015 or more, 0.02 or more, 0.025 or more, or 0.03 or more, and it also may be 1 or less, 0.95 or less, 0.9 or less, 0.85 or less, 0.8 or less, or 0.75 or less. More concretely, the molar ratio of the anionic polymer compound to 1 mole of the cationic compound contained in the first chamber may be 0.01 to 1, or 0.02 to 0.9, or 0.03 to 0.8, but it is not limited thereto.

In an embodiment, in order to increase the efficiency of intracellular delivery of mRNA by the kit of the present invention, the nanoparticle in the first chamber may further comprise fusogenic lipid.

In an embodiment, the amount of the fusogenic lipid contained in the nanoparticle in the first chamber may be 0.01 to 50 % by weight, and more concretely 0.1 to 10 % by weight, based on the total weight of the drug-containing nanoparticles prepared by the kit of the present invention.

In an embodiment, the fusogenic lipid is combined with the cationic compound by hydrophobic interaction, and forms a nanoparticle structure.

In an embodiment, the fusogenic lipid may be one or a combination of two or more selected from the group consisting of phospholipid, cholesterol, and tocopherol.

More concretely, the phospholipid may be one or more selected from the group consisting of phosphatidylethanolamine (PE), phosphatidylcholine (PC) and phosphatidic acid. The phosphatidylethanolamine (PE), phosphatidylcholine (PC) and phosphatidic acid may be in a form combined with one or two C 10-24 fatty acids. Said cholesterol and tocopherol include analogues, derivatives and metabolites of each of the cholesterol and tocopherol.

Still more concretely, the fusogenic lipid may be one or a combination of two or more selected from the group consisting of dilauroyl phosphatidylethanolamine, dimyristoyl phosphatidylethanolamine, dipalmitoyl phosphatidylethanolamine, distearoyl phosphatidylethanolamine, dioleoyl phosphatidylethanolamine (DOPE), 1,2-dipalmitoleoyl-sn-glycero-3-phosphoethanolamine (DPPE), dilinoleoyl phosphatidylethanolamine, 1-palmitoyl-2-oleoyl phosphatidylethanolamine, 1,2-diphytanoyl-3-sn-phosphatidylethanolamine, 1,2-dipalmitoleoyl-sn-glycero-3-phosphocholine (DPPC), 1,2-dioleoyl-sn-glycero-3-phosphocholine (DOPC), dilauroyl phosphatidylcholine, dimyristoyl phosphatidylcholine, dipalmitoyl phosphatidylcholine, distearoyl phosphatidylcholine, dioleoyl phosphatidylcholine, dilinoleoyl phosphatidylcholine, 1-palmitoyl-2-oleoyl phosphatidylcholine, 1,2-diphytanoyl-3-sn-phosphatidylcholine, dilauroyl phosphatidic acid, dimyristoyl phosphatidic acid, dipalmitoyl phosphatidic acid, distearoyl phosphatidic acid, dioleoyl phosphatidic acid, dilinoleoyl phosphatidic acid, 1-palmitoyl-2-oleoyl 1-palmitoyl-2-oleoyl phosphatidic acid, 1,2-diphytanoyl-3-sn-phosphatidic acid, cholesterol and tocopherol.

Still more concretely, the fusogenic lipid may be one or more selected from the group consisting of dioleoyl phosphatidylethanolamine (DOPE), 1,2-dipalmitoleoyl-sn-glycero-3-phosphocholine (DPPC), 1,2-dioleoyl-sn-glycero-3-phosphocholine (DOPC) and 1,2-dipalmitoleoyl-sn-glycero-3-phosphoethanolamine (DPPE).

In an embodiment, among the components in the drug-containing nanoparticle prepared by the kit of the present invention, the amount of the anionic polymer compound may be, based on 1 part by weight of the cationic compound, 0.01 part by weight or more, 0.03 part by weight or more, 0.05 part by weight or more, 0.07 part by weight or more, 0.1 part by weight or more, 0.12 part by weight or more, 0.15 part by weight or more, 0.2 part by weight or more, 0.3 part by weight or more, 0.4 part by weight or more, or 0.5 part by weight or more, and it also may be 15 parts by weight or less, 10 parts by weight or less, 9 parts by weight or less, 8 parts by weight or less, 7 parts by weight or less, 6 parts by weight or less, 5 parts by weight or less, 4 parts by weight or less, 3 parts by weight or less, or 1 part by weight or less. In addition, the amount of the anionic polymer compound may be adjusted within the above range according to the drug. For example, in case where the drug is virus, the amount of the the anionic polymer compound may be 3 to 15 parts by weight, based on 1 part by weight of the cationic compound, and in other embodiment, in case where the drug is nucleic acid, the amount of the the anionic polymer compound may be 0.01 to 15 parts by weight, 0.05 to 15 parts by weight, 0.07 to 10 parts by weight, 0.1 to 5 parts by weight, 0.1 to 3 parts by weight, or 0.15 to 1 part by weight, based on 1 part by weight of the cationic compound.

In an embodiment, the first chamber and/or the second chamber may further comprise aqueous solution, water miscible organic solvent, or a combination thereof. The "aqueous solution" may be used for the same meaning as water solution, and may mean, for example, water, sterilized water, buffer solution, injection solution, etc., and it may be a buffer solution further comprising organic acid. The aqueous solution may be, for example, citric acid buffer, PBS buffer, etc., but it is not limited thereto. The "water miscible organic solvent" may be C1 to C4 lower alcohol, acetone, acetonitrile, a water mixture thereof or a mixture thereof, but it is not limited thereto.

In an embodiment, the cationic compound and the anionic polymer compound may be used for the nanoparticle preparation in the form of solution filtered one or more times. More concretely, the filtering may be conducted by using a hydrophilic filter. The material of the hydrophilic filter may be, for example, nylon, mixed cellulose ester (MCE), polyethylsulfone (PES), polyvinylidene difluoride (PVDF), cellulose acetate (CA), polytetrafluoroethylene (PTFE), or a mixture thereof, but it is not limited thereto. In case of being subjected to hydrophilic filtering, the drug may be included in nanoparticle more successfully, and the stability of the drug-containing nanoparticle may increase.

In an embodiment, the second chamber may further comprise a stabilizing agent suitable for improving stability of the drug. The stabilizing agent may include pH adjusting agent, inorganic salt, saccharide, surfactant, chelating agent, etc., but it is not limited thereto. The "saccharide" may mean monosaccharide, disaccharide, sugar alcohol which is reduced sugar thereof, and polymer of single or mixed polysaccharides, etc., and the polysaccharide may mean tri- or more saccharide. For example, the monosaccharide may be mannose, glucose, arabinose, fructose, galactose, etc.; the disaccharide may be sucrose, trehalose, maltose, lactose, cellobiose, gentiobiose, isomaltose, melibose, etc.; the sugar alcohol may be mannitol, sorbitol, xylitol, erythritol, maltitol, etc.; and the polysaccharide may be raffinose, dextran, starch, hydroxyethyl starch, cyclodextrin, cellulose, hetastarch, oligosaccharide, etc. but it is not limited thereto. The "pH adjusting agent" may be Tris, glycine, histidine, glutamate, succinate, phosphate, acetate, aspartate, or a combination thereof, and the "surfactant" may be sodium lauryl sulfate, dioctyl sodium sulfosuccinate, dioctyl sodium sulfonate, chenodeoxycholic acid, N-lauroylsarcosine sodium salt, lithium dodecyl sulfate, 1-octanesulfonic acid sodium salt, sodium cholate hydrate, sodium deoxycholate, glycodeoxycholic acid sodium salt, benzalkonium chloride, Triton X-100, Triton X-114, lauromacrogol 400, polyoxyl 40 stearate, polysorbate 20, 40, 60, 65 or 80, or a combination thereof, but it is not limited thereto. The "chelating agent" may be citric acid, polyphenolic acid, EDTA, DTPA, EDDHA, or a combination thereof, but it is not limited thereto. The "inorganic salt" means a salt of monovalent or divalent metal and may be NaCl, KCl, MgCl₂, CaCl₂, MgSO₄, CaSO₄, CaCO₃, MgCO₃, etc., but it is not limited thereto.

For example, in case where the drug is virus, the second chamber may further comprise 5 to 15 mM Tris, 5 to 15 mM histidine, 50 to 90 mM NaCl, 2 to 8% (w/v) sucrose, 0.5 to 1.5 mM MgCl₂, 0.005 to 0.05% (w/v) PS-80, 0.05 to 0.15 mM EDTA, and 0.1 to 1.0% (v/v) ethanol, and the pH may be 7.0 to 8.0. In other embodiment, in case where the drug is nucleic acid, the second chamber may further comprise PBS buffer, for example, a solution comprising 2.0 to 3.5 mM KCl, 1.0 to 2.5 mM KH₂PO₄, 125 to 145 mM NaCl and 7.5 to 9.5 mM Na₂HPO₄ with PH 7.0 to pH 8.0.

In an embodiment, the kit for preparing drug-containing nanoparticles of the present invention may consist of: the drug which is effective ingredient selected from nucleic acid, polypeptide, virus or combination thereof; the cationic compound; the anionic polymer compound; solvent; and one or more additives selected from pH adjusting agent, inorganic salt, saccharide, surfactant, chelating agent, or a combination thereof, wherein the anionic polymer compound has at least one acid functional group.

In an embodiment, the particle size of the drug-containing nanoparticles can be defined by Z-average value, and for example, it may be 800 nm or less, 600 nm or less, 500 nm or less, or 400 nm or less, and also may be 10 nm or more, 50 nm or more, 100 nm or more, 150 nm or more, or 200 nm or more. In a concrete embodiment, the particle size of the drug-containing nanoparticles defined by Z-average value may be, for example, 100 to 800 nm, 100 to 600 nm, 100 to 500 nm, 100 to 400 nm, or 200 to 400 nm.

The "chamber" may be any one suitable for containing the materials of nanoparticle or solvent comprising them such as glass, plastic, paper, pack, etc., but it is not limited thereto.

### [The second aspect: Nanoparticle composition for drug delivery]

The nanoparticle composition for drug delivery according to the second aspect of the present invention comprises nanoparticle, wherein the nanoparticle comprises cationic compound and anionic polymer compound, wherein the nanoparticle does not comprise drug, and wherein the anionic polymer compound has at least one acid functional group.

In an embodiment, the particle size of the nanoparticles can be defined by Z-average value, and for example, it may be 800 nm or less, 600 nm or less, 500 nm or less, 400 nm or less, 300 nm or less, 200 nm or less, or 180 nm or less, and also may be 10 nm or more, 50 nm or more, or 100 nm or more. In a concrete embodiment, the particle size of the nanoparticles defined by Z-average value may be, for example, 10 to 800 nm, 10 to 600 nm, 10 to 500 nm, or 10 to 400 nm.

The "Z-average" may mean the average of hydrodynamic diameter of particle distribution measured by using Dynamic Light Scattering (DSL). The nanoparticles have a monodisperse particle distribution, and the polydispersity index thereof may be, for example, 0.05 to 0.8, 0.1 to 0.7, or 0.2 to 0.6.

Also, in an embodiment, the surface charge of the nanoparticles may be, for example, -50 mV or more, -45 mV or more, -40 mV or more, or -35 mV or more, and also may be 40 mV or less, 30 mV or less, 20 mV or less, 10 mV or less, or 0 mV or less. In a concrete embodiment, the surface charge of the nanoparticles may be, for example, -50 to 40 mV, -45 to 30 mV, -40 to 20 mV, -40 to 10 mV, or -35 to 0 mV. The surface charge may be measured in an environment close to biological environment, and for example, it may be measured in 8 to 12 mM HEPES buffer (pH 7.0 to 7.5).

In case of maintaining the particle size and surface charge of the nanoparticles to the above level, it is preferable in terms of stability of nanoparticle structure, amounts of the components and absorbability in the body, and easiness of sterilization. For example, in case where the drug is nucleic acid, the one or more ends of the nucleic acid may be modified with one or more selected from the group consisting of cholesterol, tocopherol and fatty acids having 10 to 24 carbon atoms. The cholesterol, tocopherol and fatty acids having 10 to 24 carbon atoms include each analogue, derivative and metabolite of the cholesterol, tocopherol and fatty acids.

In a concrete embodiment, the cationic compound may be cationic lipid or cationic polymer, and more concretely, it may be cationic lipid. Such a cationic compound is the same as concretely explained above in the first aspect of the present invention.

In an embodiment, the anionic polymer compound may be anionic amphiphilic block copolymer, anionic hydrophilic polymer, anionic hydrophobic polymer, or combination thereof. Such an anionic polymer compound is the same as concretely explained above in the first aspect of the present invention.

In an embodiment, the acid functional group may be derived from an acid selected from the group consisting of inorganic acid, sulfonic acid, carboxylic acid and combinations thereof. Such an acid is the same as concretely explained above in the first aspect of the present invention.

In an embodiment, the amount of the anionic polymer compound in the nanoparticle comprised in the nanoparticle composition for drug delivery of the present invention may be, based on 1 part by weight of the cationic compound, 0.01 part by weight or more, 0.03 part by weight or more, 0.05 part by weight or more, 0.07 part by weight or more, 0.1 part by weight or more, 0.12 part by weight or more, 0.15 part by weight or more, 0.2 part by weight or more, 0.3 part by weight or more, 0.4 part by weight or more, or 0.5 part by weight or more, and it also may be 15 parts by weight or less, 10 parts by weight or less, 9 parts by weight or less, 8 parts by weight or less, 7 parts by weight or less, 6 parts by weight or less, 5 parts by weight or less, 4 parts by weight or less, 3 parts by weight or less, or 1 part by weight or less.

More concretely, the amount of the anionic polymer compound may be 0.01 to 15 parts by weight, 0.05 to 15 parts by weight, 0.07 to 10 parts by weight, 0.1 to 5 parts by weight, 0.1 to 3 parts by weight, or 0.15 to 1 part by weight, based on 1 part by weight of the cationic compound.

From the nanoparticle composition for drug delivery of the present invention, drug-containing nanoparticles can be easily prepared only by simple mixing of the composition and drug. The term "simple mixing" can include all actions of "mixing," and it means that the action of mixing is not subject to any specific conditions. The mixing can be done in various manners such as dropping, vortexing, decanting, etc., but it is not limited thereto. According to an embodiment, in case of using the nanoparticle composition for drug delivery of the present invention, drug-containing nanoparticles can be formed rapidly, for example, within 1 minute, within 30 seconds, or within 15 seconds, in an amount of 90% or more, 95% or more, or 99% or more of the amount that can be formed theoretically.

In an embodiment, the cationic compound and the anionic polymer compound form nanoparticles through electrostatic interaction, and an end user can form drug-containing nanoparticles by simply mixing the formed nanoparticles and the drug. Thus, according to an embodiment, the drug-containing nanoparticles prepared by mixing of the nanoparticle composition for drug delivery of the present invention and drug can be in a form where at least a part of the drug is combined to the outside of the nanoparticle. Such a drug-containing nanoparticle structure improves stability of the drug in blood or body fluid.

In an embodiment, the drug may be selected from nucleic acid, polypeptide, virus or combination thereof. Such a drug is the same as concretely explained above in the first aspect of the present invention.

The amount of the drug may be, based on the total weight of the drug-containing nanoparticle prepared by mixing with the nanoparticle composition for drug delivery of the present invention, for example, 30 % by weight or less, 20 % by weight or less, 10 % by weight or less, 5 % by weight or less, or 1 % by weight or less, and also may be 0.001 % by weight or more, 0.01 % by weight or more, 0.05 % by weight or more, 0.1 % by weight or more, or 0.15 % by weight or more. If the amount of the drug based on the total weight of the drug-containing nanoparticle is less than the above range, the amount of nanoparticle used as delivery carrier becomes too much as compared with the drug, and thus there may be a side effect due to the nanoparticle delivery carrier. If the amount of the drug is greater than the above range, the size of the drug-containing nanoparticle becomes too large, and thus the particle stability may be lowered and the rate of loss during filter sterilization may increase. In case where the drug is virus, the drug-containing nanoparticle may comprise virus 1x10⁶ to 1x10¹⁴ VP (Virus particle), 1x10⁷ to 1x10¹³ VP, 1x10⁸ to 1x10¹² VP, or 1x10⁹ to 1x10¹¹ VP.

In an embodiment, the amount of the anionic polymer compound may be adjusted according to the drug. For example, in case where the drug is virus, the amount of the the anionic polymer compound may be 3 to 15 parts by weight, based on 1 part by weight of the cationic compound, and in other embodiment, in case where the drug is nucleic acid, the amount of the the anionic polymer compound may be 0.01 to 15 parts by weight, 0.05 to 15 parts by weight, 0.07 to 10 parts by weight, 0.1 to 5 parts by weight, 0.1 to 3 parts by weight, or 0.15 to 1 part by weight, based on 1 part by weight of the cationic compound.

In an embodiment, the amount of the cationic compound in the drug-containing nanoparticle prepared by mixing of the nanoparticle composition for drug delivery of the present invention and drug may be, based on 1 part by weight of the drug, for example, 25 parts by weight or less, 20 parts by weight or less, 15 parts by weight or less, 10 parts by weight or less, or 5 parts by weight or less, and also may be 0.5 part by weight or more, 1 part by weight or more, 1.5 parts by weight or more, 2 parts by weight or more, or 2.5 parts by weight or more. In an embodiment, the amount of the cationic compound in the drug-containing nanoparticle may be, based on 1 part by weight of the drug, 0.5 to 25 parts by weight, 1 to 20 parts by weight, 1.5 to 15 parts by weight, 2 to 10 parts by weight, or 2.5 to 5 parts by weight. Also, in case where the drug is virus, more concretely adenovirus, the amount of the cationic compound may be, based on virus 1x10¹⁰ VP, 1 µg or more, 5 µg or more, 10 µg or more, 15 µg or more, or 18 µg or more, and also may be 150 µg or less, 100 µg or less, 50 µg or less, or 30 µg or less, and for example, it may be 1 µg to 150 µg, 5 µg to 100 µg, 10 µg to 50 µg, or 15 µg to 30 µg. If the amount of the cationic compound in the drug-containing nanoparticle is less than the above range, the drug may not be contained stably in the nanoparticle. If the amount of the cationic compound is greater than the above range, the size of the drug-containing nanoparticle becomes too large, and thus the particle stability may be lowered and the rate of loss during filter sterilization may increase.

In case where the drug is nucleic acid, the cationic compound and the nucleic acid are combined together through electrostatic interaction. In an embodiment, the ratio of quantities of electric charge of the cationic compound (N) and the nucleic acid (P) (N/P: the ratio of the positive electric charge of the cationic compound to the negative electric charge of the nucleic acid) may be 0.5 or more, 0.7 or more, 0.9 or more, or 1 or more, and also may be 100 or less, 50 or less, 20 or less, or 10 or less, and for example, it may be 0.5 to 100, 0.7 to 50, 0.9 to 20, or 1 to 10. If the ratio (N/P) is less than the above range, a sufficient amount of the nucleic acid may not be contained in the nanoparticle. If the ratio (N/P) is greater than the above range, toxicity may be induced. In addition, the N/P ratio may act importantly for expression of the effective ingredient specifically in spleen.

In an embodiment, when mixing a drug (for example, mRNA) with the nanoparticle composition for drug delivery of the present invention, in order to increase the efficiency of intracellular delivery thereof, the nanoparticle comprised in the nanoparticle composition for drug delivery of the present invention may further comprise fusogenic lipid. Such a fusogenic lipid is the same as concretely explained above in the first aspect of the present invention.

In an embodiment, the amount of the fusogenic lipid contained in the nanoparticle may be 0.01 to 50 % by weight, and more concretely 0.1 to 10 % by weight, based on the total weight of the drug-containing nanoparticles prepared by mixing with a drug (for example, mRNA).

In an embodiment, the nanoparticle composition for drug delivery of the present invention may further comprise aqueous solution, water miscible organic solvent, or a combination thereof. Such aqueous solution and water miscible organic solvent are the same as concretely explained above in the first aspect of the present invention.

In an embodiment, the cationic compound and the anionic polymer compound may be used for the nanoparticle preparation in the form of solution filtered one or more times. More concretely, the filtering may be conducted by using a hydrophilic filter. Such a hydrophilic filter is the same as concretely explained above in the first aspect of the present invention.

In an embodiment, the nanoparticle composition for drug delivery of the present invention may further comprise a stabilizing agent suitable for improving stability of the drug. Such a stabilizing agent is the same as concretely explained above in the first aspect of the present invention.

For example, in case where the drug to be mixed is virus, the nanoparticle composition for drug delivery of the present invention may further comprise 5 to 15 mM Tris, 5 to 15 mM histidine, 50 to 90 mM NaCl, 2 to 8% (w/v) sucrose, 0.5 to 1.5 mM MgCl₂, 0.005 to 0.05% (w/v) PS-80, 0.05 to 0.15 mM EDTA, and 0.1 to 1.0% (v/v) ethanol, and the pH may be 7.0 to 8.0. In other embodiment, in case where the drug to be mixed is nucleic acid, the nanoparticle composition for drug delivery of the present invention may further comprise PBS buffer, for example, a solution comprising 2.0 to 3.5 mM KCl, 1.0 to 2.5 mM KH₂PO₄, 125 to 145 mM NaCl and 7.5 to 9.5 mM Na₂HPO₄ with PH 7.0 to pH 8.0.

In an embodiment, the nanoparticle composition for drug delivery may consist of: the cationic compound; the anionic polymer compound; solvent; and one or more additives selected from pH adjusting agent, inorganic salt, saccharide, surfactant, chelating agent, or a combination thereof, wherein the anionic polymer compound has at least one acid functional group.

In an embodiment, the particle size of the drug-containing nanoparticles prepared by mixing of the nanoparticle composition for drug delivery of the present invention and drug can be defined by Z-average value, and for example, it may be 800 nm or less, 600 nm or less, 500 nm or less, or 400 nm or less, and also may be 10 nm or more, 50 nm or more, 100 nm or more, 150 nm or more, or 200 nm or more. In a concrete embodiment, the particle size of the drug-containing nanoparticles defined by Z-average value may be, for example, 100 to 800 nm, 100 to 600 nm, 100 to 500 nm, 100 to 400 nm, or 200 to 400 nm.

The present invention will be explained below in more detail with reference to the following Examples. However, the Examples are only to illustrate the invention, and the scope of the present invention is not limited thereby in any manner.

### EXAMPLES

### Example 1

### (1) Preparation of nanoparticle composition for drug delivery

A 10 mL nanoparticle composition for drug delivery was prepared. The prepared nanoparticle composition for drug delivery corresponds to the first chamber in the first aspect of the present invention.

The nanoparticle composition for drug delivery was prepared by using 1,6-dioleoyl triethylenetetramide (dioTETA) as a cationic compound, and an anionic polymer compound (mPEG-PLA-COOH) which is a block copolymer comprising monomethoxypolyethylene glycol (number average molecular weight: 2000 g/mol) as a hydrophilic block and polylactide (number average molecular weight: 1300 g/mol) as a hydrophobic block, wherein the hydrophobic block has a carboxylic acid group derived from succinic acid.

Concretely, dioTETA and mPEG-PLA-COOH were first mixed at a molar ratio of 1/0.03 (moles of dioTETA/moles of mPEG-PLA-COOH), and then water free of ribonuclease (RNase-free water) and sucrose as a cryoprotectant were added to the concentration of 4% (40 mg/mL) in the final solution. The components of the prepared nanoparticle composition for drug delivery and amounts thereof are shown in Table 1 below.

### (2) Preparation of mRNA solution

An mRNA solution was prepared by diluting mTrp2 (murine tyrosine-related protein-2) mRNA with PBS. 5 µg of mRNA was used in preparing mRNA-nanoparticles. The prepared mRNA solution corresponds to the second chamber in the first aspect of the present invention.

### (3) Preparation of mRNA-containing nanoparticles

The above-prepared nanoparticle composition and mRNA solution (corresponding to the first chamber and the second chamber in the first aspect, respectively) were mixed to prepare the final drug (Drug Product, DP) comprising mRNA-containing nanoparticles (N/P ratio=2.5). The components of the prepared final drug (DP) and amounts thereof are shown in Table 1 below.

**[Table 1]**

| | **Nanoparticle composition for drug delivery (10 mL)** | | **DP (0.5 mL)** | |
|---|---|---|---|---|
| **Components** | Amount (mg) | Concentration (mg/mL) | Amount (mg) | Concentration (mg/mL) |
| **dioTETA** | 24.27 | 2.43 | 0.253 | 0.506 |
| **mPEG-PLA-COOH** | 3.77 | 0.38 | 0.039 | 0.079 |
| **Sucrose** | 400 | 40 | 54.17 | 108.3 |
| **mRNA** | - | - | 0.1 | 0.2 |

After mixing the nanoparticle composition for drug delivery and the mRNA solution, in order to confirm whether nanoparticles were formed, the particle size (Size), particle size distribution (PDI), and surface charge (Zeta potential) were measured using a particle size analyzer (Dynamic Light Scattering, DLS), and the results are shown in Table 2 below. According to Table 2, it was confirmed that particles were uniformly formed even after simple mixing.

**[Table 2]**

| | Particle size (nm) | Particle size distribution (PDI) | Surface charge (mV) |
|---|---|---|---|
| Example 1 | 330.2 ± 18.1 | 0.4 ± 0.04 | -8.8 ± 0.8 |

### (4) Experiment to confirm whether mRNA-containing nanoparticles were prepared

In order to confirm whether the nanoparticles and mRNA were combined normally when the nanoparticle composition for drug delivery and the mRNA solution were mixed, agarose gel electrophoresis was conducted. After the simple mixing of the nanoparticle composition and the mRNA solution, a volume corresponding to 400 ng of mRNA was taken therefrom and mixed with loading dye. Then, it was loaded on 1% agarose gel and subjected to electrophoresis with suitable voltage and time, and the mRNA band image was observed through UV transilluminator. At that time, Dyne Gel Safe Red Kit (Dyne Bio) was used as nucleic acid dye.

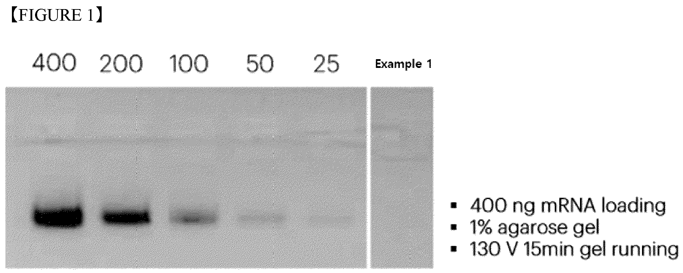

The results of the measurement are shown in Figure 1. According to Figure 1, in Example 1, the exposed mRNA band was hardly shown due to the high bonding force between the nanoparticle and mRNA.

### (5) Experiment to confirm the immune response induction effect of the formulation

In the formulation evaluation screening, the expression level of the reporter protein was utilized using luciferase encoding mRNA. In order to evaluate whether the prepared drug delivery formulation (nanoparticle composition for drug delivery-mRNA formulation) actually delivers the drug to the spleen and induces an immune response, and to confirm the immune response effect to Trp2 (Tyrosine-related protein 2) antigen, which is an important factor in melanoma, the efficacy of the formulation was evaluated through IFN-γ enzyme linked immunospot (ELISPOT) using murine Trp2 antigen derived from mouse.

For the nanoparticle composition for drug delivery-mRNA formulation, the nanoparticle composition for drug delivery prepared in Example 1 was used. As a control, an LPX (lipoplex) formulation was used. A liposome was prepared by mixing cationic lipid DOTMA (1,2-di-O-octadecenyl-3-trimethylammonium propane) and Helper lipid DOPE (dioleoylphosphatidylethanolamine), and then a liposome-mRNA (lipoplex) formulation was prepared (Nature, 2016, Vol. 534, pages 396-401).

Each of the nanoparticle composition for drug delivery-mRNA formulation and LPX (i.v. control) as a control was systemically administered to mouse, and 7 days after the administration, tissues were taken and cells were extracted and analyzed to compare the immune reactivity. The immune reactivity according to the administration dose was observed by changing the administration dose to 10, 20, and 40 µg.

The results are shown in Table 3 below. According to Table 3, the nanoparticle composition for drug delivery-mRNA formulation showed a distinct tendency according to the administration amount, and it was confirmed that the immune reactivity was higher than the control group at all administration doses.

**[Table 3]**

| | IFN-γ secreting cells per 5x10⁵ splenocytes | |
|---|---|---|
| mRNA dose (*µ*g) | Nanoparticle composition for drug delivery-mRNA | LPX |
| 10 | 168 | 62 |
| 20 | 325 | 62 |
| 40 | 590 | 72 |

### Examples 2 to 5 and Comparative Examples 1 to 3

### (1) Preparation of nanoparticle composition for drug delivery

Nanoparticle compositions for drug delivery were prepared according to the compositional details shown in Table 4 below. The prepared nanoparticle compositions for drug delivery correspond to the first chamber in the first aspect of the present invention.

Concretely, 1,6-dioleoyl triethylenetetramide (dioTETA) was dissolved in 20 mM sodium acetate buffer to prepare dioTETA solution with 5 mg/mL concentration, and the anionic polymer compound was dissolved in sterilized water to the concentration described in Table 4. Each of the solutions was filtered and sterilized by using 0.22 µm hydrophilic filter, and then the solutions were mixed with the ratio described in Table 5 below. Then, sterilized water and sucrose as a cryoprotectant were added to the concentration of 4% (40 mg/mL) in the final solution to prepare the nanoparticle composition for drug delivery. The amounts and final concentrations of the prepared nanoparticle compositions for drug delivery are shown in Table 5 below.

**[Table 4]**

| | **Cationic compound** | **Anionic polymer compound and dissolution concentration thereof** | |
|---|---|---|---|
| Example 2 | dioTETA | mPEG-PCL-COOH (2K-0.8K) | 10 mg/mL |
| Example 3 | | mPEG-PCL-COOH (2K-1.5K) | 10 mg/mL |
| Example 4 | | mPEG-COOH (2K) | 20 mg/mL |
| Example 5 | | PEOz-COOH (5K) | 50 mg/mL |
| Comparative Example 1 | | - | - |
| Comparative Example 2 | | mPEG-PLA (2K-1.7K) | 50 mg/mL |
| Comparative Example 3 | | PLA (1.7K) | 10 mg/mL |

**[Table 5]**

| | dioTETA/ Anionic polymer compound (mole) | Nanoparticle composition for drug delivery (10 mL) | | | | DP (mRNA 100 µg/0.5 mL) | | | |
|---|---|---|---|---|---|---|---|---|---|
| | | dioTETA | | Anionic polymer compound | | dioTETA | | Anionic polymer compound | |
| | | Amount (mg) | Concentration (mg/mL) | Amount (mg) | Concentration (mg/mL) | Amount (mg) | Concentration (mg/mL) | Amount (mg) | Concentration (mg/mL) |
| Example 2 | 1/0.03 | 24.27 | 2.43 | 3.02 | 0.30 | 0.233 | 0.465 | 0.029 | 0.058 |
| Example 3 | 1/0.03 | | | 3.77 | 0.38 | | | 0.036 | 0.072 |
| Example 4 | 1/0.75 | | | 53.92 | 5.39 | 0.222 | 0.445 | 0.494 | 0.989 |
| Example 5 | 1/0.75 | | | 137.51 | 13.75 | | | 1.261 | 2.521 |
| Comparative Example 1 | - | | | - | - | | | - | - |
| Comparative Example 2 | 1/0.75 | | | 99.76 | 9.98 | | | 0.914 | 1.829 |
| Comparative Example 3 | 1/0.75 | | | 45.84 | 4.58 | | | 0.420 | 0.840 |

### (2) Preparation of mRNA solution

An mRNA solution was prepared by diluting 20 µg Luciferase mRNA (CleanCap^{®} FireFly Luciferase mRNA, 5-methoxyuridine, TriLink, Catalog L-7202) with phosphate buffer. The prepared mRNA solution corresponds to the second chamber in the first aspect of the present invention.

### (3) Preparation of mRNA-containing nanoparticles

The above-prepared nanoparticle composition for drug delivery and mRNA solution were mixed by simple pipet mixing to prepare the final drug (Drug Product, DP) comprising mRNA-containing nanoparticles (N/P ratio=2.5). The components of the prepared final drug (DP) and amounts thereof are shown in Table 5 above.

After mixing the nanoparticle composition for drug delivery and the mRNA solution, in order to confirm whether nanoparticles were formed, the particle size (Size), particle size distribution (PDI), and surface charge (Zeta potential) were measured using a particle size analyzer (Dynamic Light Scattering, DLS), and the results are shown in Table 6 below. According to Table 6, it was confirmed that particles were stably formed even after simple mixing.

For Comparative Examples, nanoparticles using cationic compound only without addition of polymer (Comparative Example 1), nanoparticles using added amphiphilic polymer (mPEG-PLA (2k-1.7k)) with no anionic carboxylic acid (Comparative Example 2), and nanoparticles using added hydrophobic polylactide (PLA 1.7k) only without hydrophilic block (Comparative Example 3) were used.

**[Table 6]**

| | **Particle size (nm)** | **Particle size distribution (PDI)** | **Surface charge (mV)** |
|---|---|---|---|
| Example 2 | 188.7 (±0.8) | 0.110 (±0.003) | -27.6 (±0.9) |
| Example 3 | 182.0 (±1.6) | 0.116 (±0.011) | -20.1 (±0.2) |
| Example 4 | 260.3 (±0.6) | 0.213 (±0.011) | -53.2 (±1.4) |
| Example 5 | 331.1 (±3.9) | 0.192 (±0.012) | -31.6 (±1.3) |
| Comparative Example 1 | 333.8 (±2.5) | 0.228 (±0.005) | -45.8 (±0.2) |
| Comparative Example 2 | 266.1 (±4.7) | 0.188 (0.013) | -31.6 (±1.3) |
| Comparative Example 3 | 301.3 (±1.4) | 0.250 (±0.026) | -23.81 (±0.8) |

### (4) Experiment to confirm whether mRNA-containing nanoparticles were prepared

In order to confirm whether the nanoparticles and mRNA were combined normally when the nanoparticle composition for drug delivery and the mRNA solution were mixed, agarose gel electrophoresis was conducted. After the simple mixing of the nanoparticle composition and the mRNA solution, a volume corresponding to 500 ng of mRNA was taken therefrom and mixed with loading dye. Then, it was loaded on 1% agarose gel and subj ected to electrophoresis with suitable voltage and time, and the mRNA band image was observed through UV transilluminator. At that time, Dyne Gel Safe Red Kit (Dyne Bio) was used as nucleic acid dye.

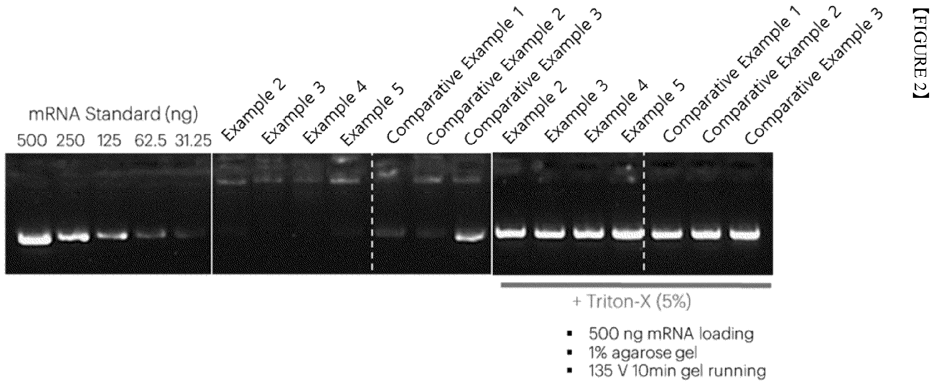

The results of the measurement are shown in Figure 2. According to Figure 2, in Examples 2 to 5, the exposed mRNA bands were hardly shown due to the high bonding force between the nanoparticle and mRNA. In contrast, in Comparative Examples 1 to 3, the exposed mRNA bands were observed due to the relatively low binding force with mRNA.

## Claims

1. A kit for preparing drug-containing nanoparticles, comprising:
a first chamber comprising nanoparticles comprising cationic compound and anionic polymer compound; and
a second chamber comprising drug which is effective ingredient selected from nucleic acid, polypeptide, virus or combination thereof,
wherein the anionic polymer compound has at least one acid functional group.

2. The kit for preparing drug-containing nanoparticles of Claim 1, wherein the drug-containing nanoparticles are for intracellular delivery of the drug.

3. The kit for preparing drug-containing nanoparticles of Claim 1**,** wherein the anionic polymer compound is anionic amphiphilic block copolymer, anionic hydrophilic polymer, anionic hydrophobic polymer, or combination thereof.

4. The kit for preparing drug-containing nanoparticles of Claim 3, wherein
the anionic amphiphilic block copolymer comprises hydrophilic block and hydrophobic block;
the anionic hydrophilic polymer comprises hydrophilic block only; and
the anionic hydrophobic polymer comprises hydrophobic block only.

5. The kit for preparing drug-containing nanoparticles of Claim 4, wherein
the hydrophilic block is one or more selected from the group consisting of polyalkylene glycol, polyvinyl alcohol, polyvinylpyrrolidone, polyoxazoline, polyacrylamide, and derivatives thereof, and
the hydrophobic block is one or more selected from the group consisting of polyester, polyanhydride, polyamino acid, polyorthoester and polyphosphazine.

6. The kit for preparing drug-containing nanoparticles of Claim 4, wherein
the hydrophilic block is one or more selected from the group consisting of monomethoxypolyethylene glycol, monoacetoxypolyethylene glycol, polyethylene glycol, polyethyloxazoline, polymethyloxazoline, copolymer of polyethylene and propylene glycol, and polyvinylpyrrolidone; and
the hydrophobic block is one or more selected from the group consisting of polylactide, polyglycolide, polycaprolactone, polydioxan-2-one, copolymer of polylactide and polyglycolide, copolymer of polylactide and polydioxan-2-one, copolymer of polylactide and polycaprolactone, and a copolymer of polyglycolide and polycaprolactone.

7. The kit for preparing drug-containing nanoparticles of Claim 1, wherein the acid functional group is derived from an acid selected from the group consisting of inorganic acid, sulfonic acid, carboxylic acid and combinations thereof.

8. The kit for preparing drug-containing nanoparticles of Claim 7, wherein
the inorganic acid is selected from the group consisting of phosphoric acid, nitric acid, chromic acid, and combinations thereof;
the sulfonic acid is selected from the group consisting of methanesulfonic acid, ethanesulfonic acid, benzenesulfonic acid, and combinations thereof; and
the carboxylic acid is selected from the group consisting of acetic acid, citric acid, gluconic acid, lactic acid, oxalic acid, tartaric acid, succinic acid, maleic acid, phthalic acid, and combinations thereof.

9. The kit for preparing drug-containing nanoparticles of Claim 1, wherein one or more selected from the group consisting of the first chamber and the second chamber further comprise additional solvent.

10. The kit for preparing drug-containing nanoparticles of Claim 9, wherein the solvent is aqueous solvent, water miscible solvent, or a mixture thereof.

11. The kit for preparing drug-containing nanoparticles of Claim 1, wherein the second chamber further comprises one or more additives selected from pH adjusting agent, inorganic salt, saccharide, surfactant, chelating agent, or a combination thereof.

12. The kit for preparing drug-containing nanoparticles of Claim 1, which consists of: the drug which is effective ingredient selected from nucleic acid, polypeptide, virus or combination thereof; the cationic compound; the anionic polymer compound; solvent; and one or more additives selected from pH adjusting agent, inorganic salt, saccharide, surfactant, chelating agent, or a combination thereof.

13. The kit for preparing drug-containing nanoparticles of Claim 1, wherein the amount of the anionic polymer compound is 0.01 to 15 parts by weight, based on 1 part by weight of the cationic compound.

14. The kit for preparing drug-containing nanoparticles of Claim 1, wherein the cationic compound and the anionic polymer compound are used for the nanoparticle preparation in the form of solution filtered one or more times.

15. A nanoparticle composition for drug delivery which is a composition for drug delivery comprising nanoparticle,
wherein the nanoparticle comprises cationic compound and anionic polymer compound, wherein the nanoparticle does not comprise drug, and
wherein the anionic polymer compound has at least one acid functional group.

16. The nanoparticle composition for drug delivery of Claim 15, wherein the anionic polymer compound is anionic amphiphilic block copolymer, anionic hydrophilic polymer, anionic hydrophobic polymer, or combination thereof.

17. The nanoparticle composition for drug delivery of Claim 15, wherein the acid functional group is derived from an acid selected from the group consisting of inorganic acid, sulfonic acid, carboxylic acid and combinations thereof.

18. The nanoparticle composition for drug delivery of Claim 15, which consists of: the cationic compound; the anionic polymer compound; solvent; and one or more additives selected from pH adjusting agent, inorganic salt, saccharide, surfactant, chelating agent, or a combination thereof.

19. The nanoparticle composition for drug delivery of Claim 15, wherein the amount of the anionic polymer compound may be 0.01 to 15 parts by weight, based on 1 part by weight of the cationic compound.

20. The nanoparticle composition for drug delivery of Claim 15, wherein the cationic compound and the anionic polymer compound are used for the nanoparticle preparation in the form of solution filtered one or more times.
